# EUROPÄISCHE PATENTANMELDUNG

(11) **EP 2 886 059 A1**
(43) Veröffentlichungstag der Anmeldung: **24.06.2015**
(21) Anmeldenummer: 13185909.2
(22) Anmeldetag: 25.09.2013
(51) Int. Cl.: A61B 8/08, G06T 7/00, G06T 7/20

(54) **4D-PULSKORREKTUR MIT DEFORMIERBARER REGISTRIERUNG**

(71) Anmelder: CureFab Technologies GmbH, 80637 München (DE)
(72) Erfinder: Wittmeier, Sebastian, 80634 München (DE); Runyan, Brent J., 80799 München (DE)

(57) **Zusammenfassung**

Die vorliegende Erfindung betrifft ein Verfahren zur Auswertung von Sonografiedaten zur verbesserten Darstellung eines dynamischen Objektes. Die Bewegung kann eine zyklische Bewegung sein, die z.B. durch Puls oder Atmung definiert ist. Die Sonografiedaten weisen mindestens einen ersten und einen zweiten Datensatz auf, die Daten von zwei verschiedenen Zeitpunkten umfassen und räumlich überlappen. Durch Bestimmen mindestens einer Markierung im ersten und zweiten Datensatz und durch Ermitteln einer Bewegung und/oder Deformation, die die Markierung im ersten Datensatz in die Markierung im zweiten Datensatz transformiert, wird die Bewegung und/oder Deformation des Objekts ermittelt und eine Sonografiedatenkorrektur abgeleitet. Aus den Deformationsinformationen lässt sich über Interpolation, Filterung und/oder Glättung ein optimiertes Deformationstensorfeld erzeugen, mithilfe dessen nicht vorhandene Datensätze interpoliert werden können.

## Beschreibung

Die vorliegende Erfindung betrifft ein Verfahren zur Auswertung von Sonografiedaten zur verbesserten Darstellung eines dynamischen Objekts, ein Computerprogramm zur Durchführung eines solchen Verfahrens sowie ein Ultraschallgerät, das geeignet ist, ein solches Verfahren durchzuführen.

Bei der Sonografie wird die Daten- bzw. Bildqualität häufig dadurch limitiert, dass sich das darzustellende Objekt während der Aufnahmezeit bewegt. Dabei kann es sich um zyklische Bewegungen handeln, wenn beispielsweise eine Sonografie am schlagenden Herzen durchgeführt wird, oder auch um nicht-zyklische Bewegungen, da beispielsweise der zu untersuchende Patient nicht ruhig liegt. In manchen Fällen kann zwar eine zyklische Bewegung vorliegen, verursacht beispielsweise durch die Atmung des Patienten, ohne dass der gesamte Zyklus während der Aufnahme der Sonografiedaten durchlaufen wird, sodass die aufgenommenen Daten letztendlich eine nicht-zyklische Bewegung enthalten. In all diesen Fällen beeinträchtig die Bewegung die Bildqualität der Sonografiedaten. Im Fall zyklischer bzw. periodischer Bewegungen oder Deformationen (aufgrund von Puls oder Atmung) wird oft sogenanntes Gating eingesetzt. Das heißt über eine extern gemessene oder aus den Bilddaten bestimmte Größe wird die Phase des aktuellen Bildes bestimmt. Bei der Bilderstellung werden dann beispielsweise nur Daten einer Phase (z.B. der Endphase der Diastole oder der Endphase der Systole) verwendet und miteinander kombiniert, um ein Sonogramm zu erstellen. Hierbei kann einfaches Gating erfolgen, wobei das Sonogramm nur zu einer Phase dargestellt wird, oder es wird für unterschiedliche Phasen bzw. Phasenabschnitte zeitaufgelöst das finale Sonogramm erstellt.

Bei dieser bereits bekannten Technologie entstehen jedoch verschiedene Nachteile. Zunächst findet Gating sinnvollerweise nur bei periodischen bzw. zyklischen Bewegungen oder Deformationen Anwendung. Bildstörungen, die durch ein (zusätzliches) Bewegen des Patienten erzeugt werden, können mithilfe dieses Gatings nicht behoben werden. Darüber hinaus handelt es sich in der Realität oft nicht um perfekt periodische Bewegungen oder Deformationen. So wird beispielsweise in der Regel nicht immer gleich tief eingeatmet, sodass der Wechsel zwischen Einatmen und Ausatmen nicht notwendigerweise mit dem gleichen Deformationszustand des Körpers koordiniert.

Die vorliegende Erfindung stellt sich daher die Aufgabe, ein Verfahren zur Auswertung von Sonografiedaten bereitzustellen, dass eine verbesserte Darstellung eines dynamischen Objekts ermöglicht. Diese Aufgabe wird mit dem Verfahren gemäß Anspruch 1 gelöst. Bevorzugte Ausführungsformen der vorliegenden Erfindung sind in den abhängigen Ansprüchen beschrieben.

Erfindungsgemäß werden Sonografiedaten von einem dynamischen Objekt bereitgestellt, wobei die Sonografiedaten mindestens einen ersten und einen zweiten Datensatz aufweisen, wobei der erste Datensatz und der zweite Datensatz zumindest teilweise räumlich überlappen und wobei der erste Datensatz mindestens Sonografiedaten zu einem ersten Zeitpunkt t₁ umfasst und der zweite Datensatz mindestens Sonografiedaten zu einem zweiten, vom ersten Zeitpunkt verschiedenen Zeitpunkt t₂ umfasst. Bei dem dynamischen Objekt kann es sich um ein periodisch und/oder nicht-periodisch bewegtes und/oder deformiertes Objekt handeln. Bei dem Objekt kann es sich insbesondere um einen Teil oder einen Abschnitt eines menschlichen oder tierischen Körpers handeln. Dabei kommen alle Teile bzw. Abschnitte des Körpers infrage, die grundsätzlich für Ultraschalluntersuchungen zugänglich sind.

Erfindungsgemäß wird im ersten und zweiten Datensatz mindestens eine Markierung bestimmt und eine Bewegung und/oder Deformation ermittelt, die die Markierung im ersten Datensatz in die Markierung im zweiten Datensatz transformiert. Mit dem Begriff "Markierung" ist dabei ein bestimmtes Element oder eine Kombination von bestimmten Elementen im ersten und zweiten Datensatz gemeint, die in beiden Datensätzen identifizierbar und einander zuordenbar ist. Diese Markierung im ersten und zweiten Datensatz korrespondiert dabei mit einer entsprechenden Struktur im dynamischen Objekt.

Auf Basis der ermittelten Bewegung und/oder Deformation der mindestens einen Markierung wird dann eine Bewegung und/oder Deformation des Objekts oder zumindest eines Teils oder Abschnitts des Objekts ermittelt. Je nach Art und Komplexität der Bewegung bzw. Deformation des dynamischen Objekts sind hierfür mehrere Markierungen oder eine räumlich ausgedehnte Markierung von Vorteil. Bei einer linearen Bewegung des gesamten Objekts kann eine einzelne punktförmige Markierung ausreichend sein. Im Falle einer kontinuierlichen Deformation eines Blutgefäßes ist es beispielsweise bevorzugt, mehrere Markierungen auf der Gefäßwand zu bestimmen oder beispielsweise die gesamte Gefäßwand als Markierung zu verwenden.

Auf Basis der ermittelten Bewegung und/oder Deformation des Objekts wird dann eine Sonografiedatenkorrektur abgeleitet und unter Verwendung des ersten und/oder zweiten Datensatzes sowie dieser abgeleiteten Sonografiedatenkorrektur ein korrigiertes Sonogramm des Objekts oder zumindest eines Teils oder Abschnitts des Objekts erstellt.

Die Sonografiedatenkorrektur ist bevorzugt eine Vorschrift oder Darstellung, um zwischen verschiedenen Bewegungs- und/oder Deformationszuständen des Objekts umzurechnen. Diese kann beispielsweise modellbasiert mit einem oder mehreren Freiheitsgraden oder als Vektorfeld vorliegen.

Mit anderen Worten basiert die vorliegende Erfindung darauf, die Deformation und/oder Bewegung des dynamischen Objekts aus den Sonografiedaten heraus zu bestimmen und die aufgrund dieser Deformation und/oder Bewegung verursachten Verzerrungen und/oder Verschiebungen in den Bilddaten entsprechend zu korrigieren. Dabei wird die Bewegung und/oder Deformation des dynamischen Objekts anhand der Bewegung und/oder Deformation spezifischer struktureller Bereiche im dynamischen Objekt bestimmt, die im ersten und zweiten Datensatz identifiziert und als Markierung verwendet werden können.

Die mindestens eine Markierung kann dabei eines oder eine Kombination der folgenden Elemente aufweisen: einen oder mehrere Markierungspunkte, eine oder mehrere Markierungskanten, eine oder mehrere Markierungsflächen, eine oder mehrere Grenzflächen, insbesondere Grenzflächen zwischen unterschiedlichen Materialien des Objekts, eine oder mehrere Oberflächen von Teilobjekten des Objekts, anatomische Merkmale des Objekts, absolute und/oder relative Intensitätswerte und/oder Frequenzspektrum der Intensitätswerte mindestens eines Teils der Sonografiedaten. Streng genommen ist dabei zu unterscheiden zwischen einer Struktur, die im dynamischen Objekt selbst vorliegt, und einer, mit dieser Struktur korrespondierenden Markierung in den Sonografiedaten. So korrespondiert beispielsweise die Gefäßwand eines Blutgefäßes im dynamischen Objekt mit einer entsprechenden Markierungsfläche bzw. Grenzfläche in den Sonografiedaten. Mit dem Begriff "Markierung" ist im Kontext der vorliegenden Erfindung jeweils ein identifizierbares Element bzw. ein identifizierbarer Bereich in den Sonografiedaten selbst gemeint.

Als Markierungen können beispielsweise geometrische und/oder anatomische Merkmale sowie den Rohdaten inhärente Merkmale in Betracht kommen. Bevorzugte geometrische Merkmale sind:
- Kanten (Edges), also nahe beieinander liegende Punkte mit hohem Gradienten; es können zusätzliche Anforderungen an die Form oder Glattheit gestellt werden
- Ecken (Corners / Interest Points), meist Punkte, an denen der Gradient die Richtung ändert (also eine starke Krümmung hat)
- Regionen (Blobs / Interest Regions), größere Regionen mit ähnlichen Eigenschaften wie die Ecken
- Ridges, ähnlich wie Kanten, die durch symmetrische Spiegelung verdoppelt wurden.

Solche geometrischen Merkmale können beispielsweise mit Featurebeschreibungen wie SIFT, SURF, GLOH oder HOG standardmäßig erfasst und anschließend klassifiziert werden. Diese Techniken sind beispielsweise in den folgenden Publikationen beschrieben: Mikolajczyk, K.; Schmid, C., "A performance evaluation of local descriptors," Pattern Analysis and Machine Intelligence, IEEE Transactions on, vol.27, no.10, pp.1615-1630, 2005. Dalal, N.; Triggs, B., "Histograms of oriented gradients for human detection," Computer Vision and Pattern Recognition, 2005. CVPR 2005. IEEE Computer Society Conference on, pp.886-893 vol. 1, 2005. Lowe, D.G., "Object recognition from local scale-invariant features," Computer Vision, 1999. The Proceedings of the Seventh IEEE International Conference on, vol.2, pp.1150-1157, 1999. Bay, H.; Ess, A.; Tuytelaars, T.; Van Gool, L., "Speeded-Up Robust Features (SURF), Computer Vision and Image Understanding, Vol. 110, Issue 3, pp. 346-359, 2008.

Die geometrischen Merkmale können anhand von semantischen Informationen, also dem Wissen über das typische Aussehen bestimmter aufgenommener Objekte, so kombiniert oder parametrisiert werden, dass sie anatomische Objekte diesen Typs erkennen. Z.B. ist in Ultraschallbildern (B-Mode, ohne Kontrastmittel) das Gefäßlumen schwarz, hat gefäßabhängig eine typische Breite und darum weiße Gefäßwände. Gefäße sind relativ rund und ändern sich in einer Dimension (längs des Gefäßes) wenig. Daher kann beispielsweise das sogenannte Frangi-Verfahren zur Anwendung kommen, welches durch die Eigenwerte der zweiten Ableitungen eines Gaußfilters die Hauptachsen und die Änderungen entlang der Hauptachsen bestimmt und die Werte als Erkennungsmaß für Gefäße gewichtet (vgl. Frangi goes US: multiscale tubular structure detection adapted to 3D ultrasound; Waelkens P. et al.; Med Image Comput Comput Assist Interv. 2012;15(Pt 1):625-33). Für diese Kombination oder Parametrisierung geometrischer Merkmale kommen z. B. Gefäßwände, Herzklappen, Herzkammern und die Hautoberfläche in Frage.

Zusätzlich oder alternativ kann eine Segmentierung angewandt werden (vgl. Mitral Annulus Segmentation From Three-Dimensional Ultrasound; Schneider, Robert J. et al. 2009. In Proceedings of the 2009 6th IEEE International Symposium on Biomedical Imaging: From Nano to Macro: June 28, 2009 - July 1, 2009, Boston, MA, 779-782. Boston, MA: IEEE).

Markierungen können auch helligkeitsbasiert definiert werden. Als Vergleichskriterium werden beispielsweise direkt Helligkeitswerte des ganzen Bildes oder eines Bildausschnitts verwendet und nach einer Angleichung (Normalisierung) z. B. eines der folgenden Ähnlichkeitsmaße angewandt:
- SSD-SAD (Sum of squared differences)
- Cross Correlation (Sum of absolute differences)
- Mutual information
- Ratio Image Uniformity.

Zur deformierbaren Registrierung können auch Speckles, also Beugungsmuster des Ultraschalls oder die Wellenrohdaten verwendet werden (vgl. Registration of RF ultrasound data using hybrid local binary patterns; Klein, T. et al.; 9th IEEE International Symposium on Biomedical Imaging (ISBI), 2012; "Ultrasound Speckle Tracking For Strain Estimation"; Revell, J.; Mirmehdi, M.; McNally, D., " Ultrasound Speckle Tracking For Strain Estimation", University of Bristol, Department of Computer Science, 2003).

Eine Beschreibung von Verfahren zur Durchführung einer deformierbaren Registrierung, wie sie im Kontext der vorliegenden Erfindung verwendet werden kann, findet sich beispielsweise in dem Übersichtsartikel "Deformable Medical Image Registration: A Survey" von A. Sotiras et al. (Reserach Report No. 7919; 2012; Project Team GALEN). Grundsätzlich wird dazu in den meisten Verfahren ein Deformierungsmodell mithilfe eines Energiemodells bewertet und das Ergebnis über ein iteratives Optimierungsverfahren gefunden. Für die vorliegende Anwendung gibt es zwei bevorzugte Ansätze:
1. Ausgehend von zwei Zeitpunkten oder Phasen mit jeweils einem dazugehörigen 3D-Volumen wird die Sonografiedatenkorrektur mit deformierbarer Registrierung bestimmt. Die 3D-Daten können dabei gewonnen werden, indem
   - die Sonografiedaten dreidimensional zu zwei Zeitpunkten oder Phasen gescannt werden oder
   - 1D- oder 2D-Sonografiedaten jeweils derselben Phase, z.B. mit einer Interpolation oder einer 3D-Rekonstruktion, kombiniert werden.
2. Das Deformierungsmodell bestimmt ein 4D-Deformierungsfeld, wobei eine Dimension die Zeit oder Phase ist und die anderen drei Dimensionen die Raumdimensionen sind. Es wird nun möglicherweise aus dünnbesetzten Daten ein glattes 4D-Deformierungsfeld optimiert, welches mehrere 1D-, 2D- oder 3D-Sonografiedaten zwischen den Zeitpunkten/Phasen und zwischen den Orten überführt.

Das Ableiten der Sonografiedatenkorrektur weist bevorzugt, je nach Art der Bewegung und/oder Deformation des dynamischen Objekts, auf: Bestimmen mindestens eines Verschiebevektors mindestens eines Markierungspunktes und/oder Bestimmen mindestens einer Deformationsmatrix und/oder Bestimmen eines geglätteten Deformationsfelds. Geht man von einer "reinen" Bewegung eines im Wesentlichen nicht-deformierten Objekts aus, kann das Bestimmen eines einzelnen Verschiebevektors eines einzelnen Markierungspunktes ausreichen, wohingegen eine Deformation in der Regel die Bestimmung einer Deformationsmatrix erfordert, wobei davon ausgegangen wird, dass es sich bei der Deformation um eine lineare Transformation handelt. Bekannte Verfahren zur Durchführung solcher Deformationen sind beispielsweise in "Deformable Medical Image Registration: A Survey" von A. Sotiras et al. (Reserach Report No. 7919; 2012; Project Team GALEN) beschrieben.

Bevorzugt erfolgt das Erstellen des Sonogramms derart, dass der erste und/oder zweite Sonografiedatensatz korrigiert wird und ein Sonogramm des Objekts zu einem Referenzzeitpunkt, bevorzugt aus dem Zeitintervall [t₁, t₂] oder durch Extrapolation erstellt wird. Besonders bevorzugt entspricht der Referenzzeitpunkt dem ersten oder zweiten Zeitpunkt. Es kann aber auch ein Referenzzeitpunkt zwischen dem ersten und zweiten Zeitpunkt festgelegt werden und ein interpoliertes Sonogramm für diesen Referenzzeitpunkt erstellt werden.

Bevorzugt weisen die Sonografiedaten mehrere Datensätze auf, die jeweils unterschiedliche Zeitpunkte umfassen, wobei jeweils mindestens zwei der mehreren Datensätze zumindest teilweise räumlich überlappen. In diesem Fall werden bevorzugt mehrere korrigierte Sonogramme für mehrere Referenzzeitpunkte erstellt, indem jeweils eine Bewegung und/oder Deformation einer Markierung zwischen zwei Datensätzen ermittelt und daraus auf die Bewegung und/oder Deformation des Objekts zwischen diesen zwei Datensätzen bestimmt wird. Entsprechend kann für jeden der mehreren Zeitpunkte eine Sonografiedatenkorrektur abgeleitet werden.

Gemäß einer besonders bevorzugten Ausführungsform stammen die Sonografiedaten von einem zyklisch bewegten und/oder deformierten Objekt. Die Sonografiedaten umfassen dabei bevorzugt mindestens eine Periode der Bewegung und/oder Deformation, sodass anhand der Sonografiedaten auf die zyklische Bewegung und/oder Deformation geschlossen werden kann. Besonders bevorzugt umfassen die Sonografiedaten jedoch mindestens zwei, bevorzugt mehrere Phasen. Dabei umfasst der erste Datensatz Daten einer ersten Phase ϕ₁ der zyklischen Bewegung und/oder Deformation und der zweite Datensatz Daten einer zweiten, von der ersten Phase verschiedenen Phase ϕ₂ der zyklischen Bewegung und/oder Deformation. Das Erstellen des Sonogramms erfolgt dabei bevorzugt derart, dass der erste und/oder zweite Sonografiedatensatz korrigiert wird und ein Sonogramm des Objekts oder zumindest eines Teils oder Abschnitts des Objekts bevorzugt zu einer Referenzphase aus dem Phasenintervall [ϕ₁, ϕ₂] oder durch Extrapolation erstellt wird. Dabei ist es besonders bevorzugt, dass der erste Datensatz Daten unterschiedlicher Zeiten, aber derselben Phase ϕ₁ umfasst und/oder der zweite Datensatz Daten unterschiedlicher Zeiten, aber derselben Phase ϕ₂ umfasst. Dabei wird davon ausgegangen, dass zwischen Daten derselben Phase keine Korrektur nötig ist, sodass Daten derselben Phase, aber unterschiedlicher Zeiten zusammengefasst bzw. zur Verbesserung der Bildqualität gemittelt werden können und eine Korrektur lediglich im Bezug auf (gemittelte) Daten einer anderen Phase erfolgt.

Bevorzugt wird dabei das erfindungsgemäße Verfahren für mehrere Datensätze unterschiedlicher Phasen durchgeführt und ein Sonogramm des Objekts für mehrere Referenzphasen erstellt. Bevorzugt decken die mehreren Referenzphasen im Wesentlichen einen kompletten Zyklus der Bewegung und/oder Deformation ab.

Bevorzugt wird ferner die Phase der zyklischen Bewegung und/oder Deformation bestimmt, wobei die Phase auf Basis der Sonografiedaten und/oder mithilfe einer zusätzlichen Messeinheit bestimmt wird. Wird beispielsweise die zyklische Bewegung und/oder Deformation durch den Pulsschlag eines Patienten definiert, so kann die Bestimmung der Phase mithilfe eines EKG-Messgeräts und/oder eines Pulsoximeters erfolgen. Wird die zyklische Bewegung und/oder Deformation beispielsweise durch Atmung definiert, so kann die Phase bevorzugt mithilfe eines Spirometers bestimmt werden. In diesen Fällen umfasst der Schritt des Bereitstellens von Sonografiedaten den zusätzlichen Schritt des Bereitstellens von Daten einer oder mehrerer Messeinheiten, die Information bezüglich der Phase der zyklischen Bewegung und/oder Deformation enthalten. Eine bildbasierte Bestimmung der Phase ist im Falle der Atmung beispielsweise in "Manifold Learning for Image-Based Breathing Gating with Application to 4D Ultrasound" (Wachinger, C.; Yigitsoy, M.; Navab, N., Medical Image Computing and Computer-Assisted Intervention" MICCAI 2010, pp.26-3 3, 2010.) beschrieben.

Bevorzugt wird die Datenqualität der Sonografiedaten ermittelt und mindestens ein Teil der Sonografiedaten eines oder mehrerer Zeitpunkte bzw. einer oder mehrerer Phasen schlechter bzw. ungenügender Datenqualität verworfen. Die Datenqualität der Sonografiedaten kann dabei auf Basis der ermittelten Schärfe bzw. des ermittelten Kontrastes in axialer und/oder lateraler Richtung, mithilfe der Resultate einer sogenannten "edge detection" oder mithilfe anderer bekannter Verfahren ermittelt werden. Insbesondere können diejenigen Daten verworfen werden, die zu Zeiten schneller Bewegung und/oder Deformation aufgenommen wurden.

Bereiche mit schlechter Datenqualität können beispielsweise mit den folgenden Verfahren ermittelt werden:
- Unterschiede zwischen den beiden durch Transformation vergleichbaren Sonografiedaten. Ein Vergleich zeigt, ob die beiden Sonografiedaten sehr unterschiedlich und damit auch mindestens einer der beiden Datensätze von schlechter Qualität ist. Durch weitere Vergleiche oder andere Kriterien kann geschlossen werden, welcher Datensatz von höherer Qualität ist.
- Geringe Schärfe/Kontrast in axialer und lateraler Richtung z.B. über die Resultate einer Edge Detection oder mit Hilfe der in den folgenden Publikationen beschriebenen Verfahren: "Image Sharpness measure using eigenvalues" (C. Wee et al.; 9th International Conference on Signal Processing, 2008) "Blur Detection for Digital Images Using Wavelet Transform" (Hanghang Tong; Mingjing Li; HongJiang Zhang; Changshui Zhang, Multimedia and Expo, 2004. ICME '04. 2004 IEEE International Conference on, vol.1, no., pp.17-20 Vol.1, 2004.)
- Schnelle Bewegung (positionsabhängig) aus der Sonografiedatenkorrektur
- Große Deformation (positionsabhängig) aus der Sonografiedatenkorrektur.

Bevorzugt wird das erfindungsgemäße Verfahren für mehrere Zeitpunkte bzw. Phasen durchgeführt und mehrere Sonogramme zu diesen unterschiedlichen Zeitpunkten oder Phasen erstellt. Besonders bevorzugt werden diese mehreren Sonogramme zeitlich nacheinander angeordnet oder dargestellt.

Die vorliegende Erfindung betrifft ferner ein Computerprogramm zur Durchführung des oben beschriebenen Verfahrens.

Die vorliegende Erfindung betrifft ferner ein Ultraschallgerät mit einer Signalprozessoreinheit, wobei das Ultraschallgerät dazu geeignet ist, Sonografiedaten von einem dynamischen Objekt aufzunehmen. Die Sonografiedaten weisen mindestens einen ersten und einen zweiten Datensatz auf, wobei der erste Datensatz und der zweite Datensatz zumindest teilweise räumlich überlappen und wobei der erste Datensatz mindestens Sonografiedaten zu einem ersten Zeitpunkt t₁ und der zweite Datensatz mindestens Sonografiedaten zu einem zweiten, vom ersten Zeitpunkt verschiedenen Zeitpunkt t₂ umfasst. Dabei ist die Signalprozessoreinheit dazu geeignet, das oben beschriebene Verfahren durchzuführen.

Bevorzugt weist das Ultraschallgerät ferner eine Einrichtung zum Messen von Puls und/oder Atmung, beispielsweise ein EKG-Messgerät und/oder ein Pulsoximeter und/oder ein Spirometer, auf.

Das erfindungsgemäße Verfahren sowie das erfindungsgemäße Ultraschallgerät ermöglichen eine deutlich verbesserte Auswertung von Sonografiedaten eines dynamischen Objekts. Mithilfe des erfindungsgemäßen Verfahrens lassen sich die aufgrund von Bewegungen und/oder Deformationen des darzustellenden Objekts auftretenden Störungen korrigieren. Dabei muss nicht, wie beispielsweise in der herkömmlichen Gating-Technologie auf einen Großteil der Daten verzichtet werden. Vielmehr kann ein Großteil der Daten oder sogar alle Daten durch eine entsprechende Korrektur kombiniert werden, um so ein qualitativ besonders hochwertiges Sonogramm zu erstellen.

Nachfolgend werden besonders bevorzugte Ausführungsformen der vorliegenden Erfindung mit Bezug auf die Figuren näher beschrieben. Es zeigen:
- Fig. 1-5: schematisch verschiedene bevorzugte Ausführmgsformen des erfindungsgemäßen Verfahrens;
- Fig. 6: Flussdiagramme bevorzugter Ausführungsformen des erfmdungsgemäßen Verfahrens; und
- Fig. 7: schematisch ein Ausführungsbeispiel anhand eines Blutgefäßes.

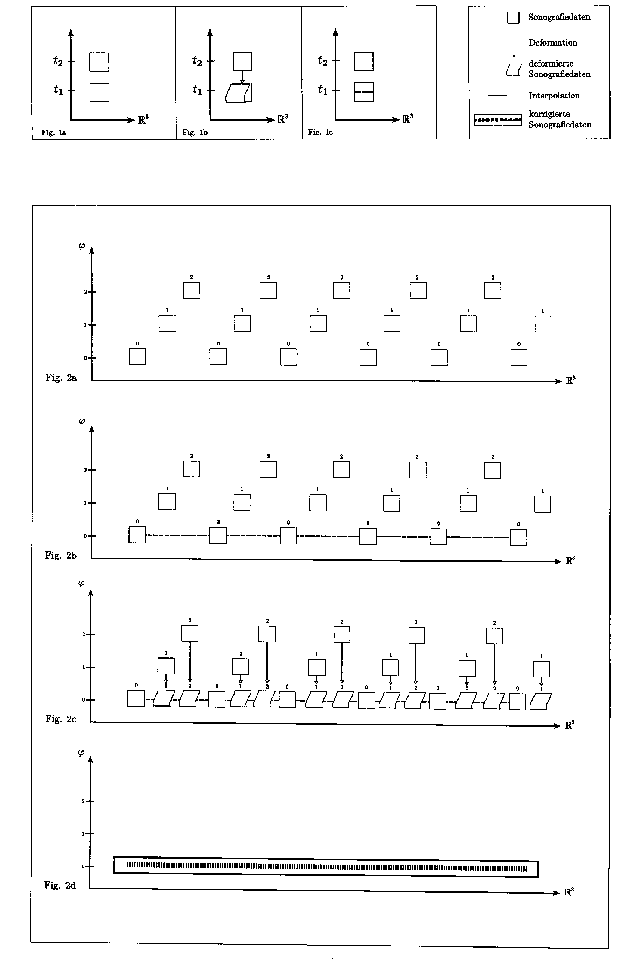

Die Figuren 1a bis 1c zeigen schematisch das Konzept des erfindungsgemäßen Verfahrens. Wie in Figur 1a zu sehen ist, werden Sonografiedaten (vgl. Legende in Figur 1 ganz rechts) von einem dynamischen Objekt bereitgestellt, wobei die Sonografiedaten mindestens einen ersten und einen zweiten Datensatz aufweisen, wobei der erste Datensatz und der zweite Datensatz zumindest teilweise räumlich überlappen und wobei der erste Datensatz mindestens Sonografiedaten zu einem ersten Zeitpunkt t₁ umfasst und der zweite Datensatz mindestens Sonografiedaten zu einem zweiten Zeitpunkt, vom ersten Zeitpunkt verschiedenen Zeitpunkt t₂ umfasst.

Durch Bestimmen mindestens einer Markierung im ersten und zweiten Datensatz und durch Ermitteln einer Bewegung und/oder Deformation, die die Markierung im ersten Datensatz in die Markierung im zweiten Datensatz transformiert (nicht dargestellt), wird die Bewegung und/oder Deformation des Objekts ermittelt (nicht dargestellt). Damit lässt sich eine Sonografiedatenkorrektur ableiten, mithilfe derer die deformierten Daten zum Zeitpunkt t₁ in korrigierte Sonografiedaten überführt werden können (vgl. Figur 1b). Auf Basis des korrigierten ersten Datensatzes sowie des zweiten Datensatzes wird dann ein korrigiertes Sonogramm des Objekts erstellt (vgl. Figur 1c).

In Figur 2 ist schematisch die Situation dargestellt, in der mithilfe eines einzelnen Scan-Schwungs ein periodisch bewegtes und/oder deformiertes Objekt gescannt wird. Der Einfachheit halber sind lediglich 5 2/3 Perioden der zyklischen Bewegung bzw. Deformation und jeweils drei Phasen 0, 1 und 2 dargestellt. Während der Schallkopf am deformierten Objekt vorbeibewegt wird, durchläuft dieser, wie in Figur 2a dargestellt, nacheinander die Phasen 0, 1, 2, 0, 1, 2, 0, 1, 2, 0, 1, 2, 0, 1, 2, 0 und 1. Da das dynamische Objekt in den Phasen 1 und 2 bezüglich der Phase 0 deformiert bzw. bewegt ist, liegt, wie in Figur 2a dargestellt, im Hinblick auf die hier ausgewählte Referenzphase 0 eine Folge von nicht deformierten und deformierten Sonografiedaten vor. Aus der Folge von nicht deformierten Sonografiedaten der Phase 0 kann über eine Interpolation oder eine 3D-Rekonstruktion ein dreidimensionales Referenzvolumen gebildet werden (Figur 2b).

Für die einzelnen deformierten Sonografiedaten der Phasen 1 und 2 kann nun jeweils durch Ermittlung einer Bewegung und/oder Deformation zum Referenzvolumen aus Phase 0 - wie bereits im Hinblick auf Figur 1 diskutiert- durch Ableiten einer Sonografiedatenkorrektur ein Satz von korrigierten Sonografiedaten erstellt werden (vgl. Figur 2c) und aus diesen wiederum - und evtl. mithilfe von Interpolation oder 3D-Rekonstruktion - ein korrigiertes Sonogramm des Objekts (Figur 2d). Dieses korrigierte Sonogramm hat eine höhere räumliche Auflösung als das Referenzvolumen, welches ausschließlich aus den Sonografiedaten der Phase 0 gebildet wurde. Figur 2d zeigt das gesamte gescannte Objekt für die Phase 0. Analog lässt sich die Korrektur für die Phasen 1 und 2 durchführen, sodass das gesamte Objekt für diese Phasen korrigiert dargestellt werden kann.

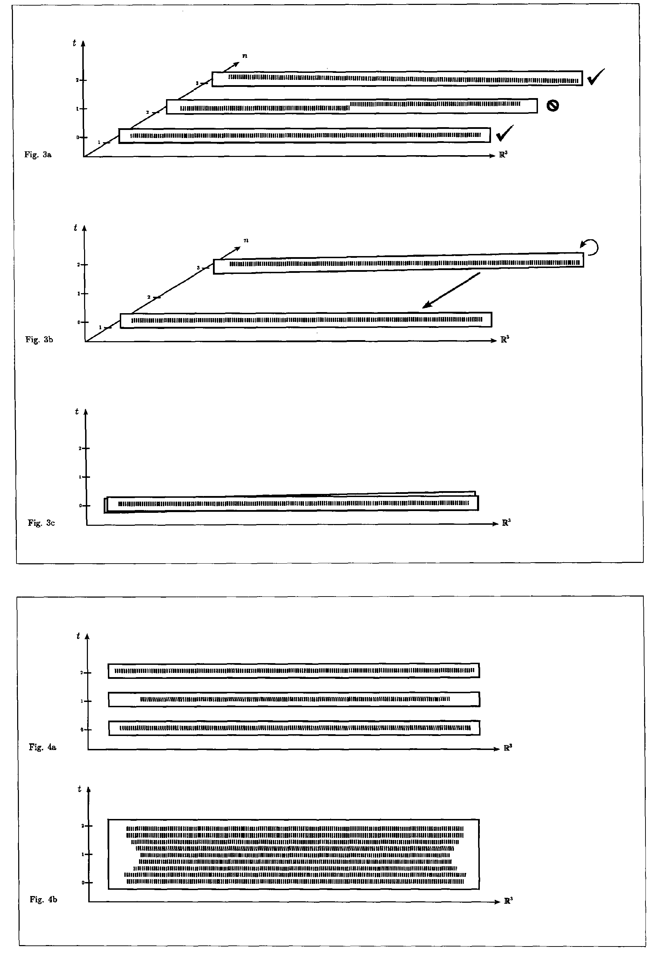

Anstelle eines einzelnen Scan-Schwungs wie in Figur 2 können auch mehrere Scan-Schwünge nacheinander erfolgen, wie dies in Figur 3 dargestellt ist. Gemäß einer bevorzugten Ausführungsform werden in diesem Fall die Daten derjenigen Scan-Schwünge, während denen der zyklischen Bewegung des Objekts eine weitere Bewegung überlagert ist (z.B. ein Patient mit pulsierendem Gefäß, der sich während einer Aufnahme bewegt), nicht verwendet. In Figur 3a sind drei Scan-Schwünge, welche jeweils z.B. durch Anwendung des Verfahrens nach Figur 2 entstanden, schematisch dargestellt, die sich jeweils paarweise unterscheiden. Scan-Schwung 1 und Scan-Schwung 3 können mit einer nichtverformenden Abbildung (hier eine Drehung) ineinander übergeführt werden. Scan-Schwung 2 kann jeweils nicht mit einer nichtverformenden Abbildung in Scan-Schwung 1 oder Scan-Schwung 3 übergeführt werden und ist damit inkonsistent mit den anderen beiden. Aus den nicht verworfenen Scan-Schwüngen wird durch Anwendung der nichtverformenden Abbildung und Kombination der beiden 3D-Volumen ein optimiertes 3D-Sonogramm erstellt (vgl. 3c).

Die nichtverformende Abbildung von Scan-Schwung 3 auf Scan-Schwung 1 kann auch auf die zugrundeliegenden Datensätze angewandt werden. Dies ist vorteilhaft, da die beiden 3D-Volumen aus Scan-Schwung 1 und Scan-Schwung 3 im Allgemeinen pro Position Daten unterschiedlicher Phasen enthalten können, die optional durch erneute Anwendung des in Figur 2 beschriebenen Verfahrens auf den kombinierten Datensatz zusammengeführt werden können (nicht dargestellt).

Durch Anwendung eines der beschriebenen Verfahren zur Erzeugung eines optimierten Sonogramms nacheinander für verschiedene Zeitpunkte als Referenzzeitpunkte oder Phasen als Referenzphase, in Figur 4a dargestellt für die Phasen oder Zeitpunkte 0, 1 und 2, kann ein Datensatz mit mehreren Zeitpunkten oder Phasen erzeugt werden, bei dem diese bevorzugt nacheinander, also 4-dimensional, angeordnet oder dargestellt werden (Figur 4b).

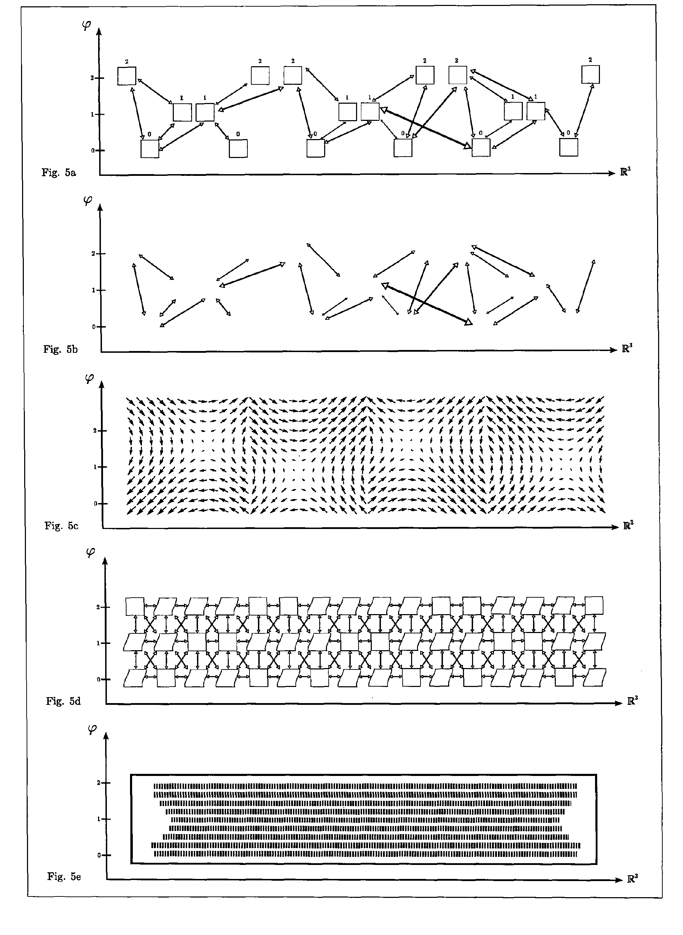

Statt wie in Figur 4 nach Ort und Zeit/Phase getrennt zusammenzuführen, können die vorhandenen Datensätze möglicherweise dünn besetzt nach Ort und Zeit oder Phase angeordnet werden und eine Deformation zu zeitlich und/oder örtlich naheliegenden Datensätzen (siehe Figur 5a) bestimmt werden. Aus diesen Deformationsinformationen (Figur 5b) lässt sich über Interpolation, Filterung und/oder Glättung ein optimiertes 4-dimensionales Deformationstensorfeld erzeugen (Figur 5c). Mithilfe dessen und der vorhandenen Datensätze können nicht vorhandene Datensätze interpoliert werden (Figur 5d) und ein vollständiger 4-dimensionaler Datensatz generiert werden.

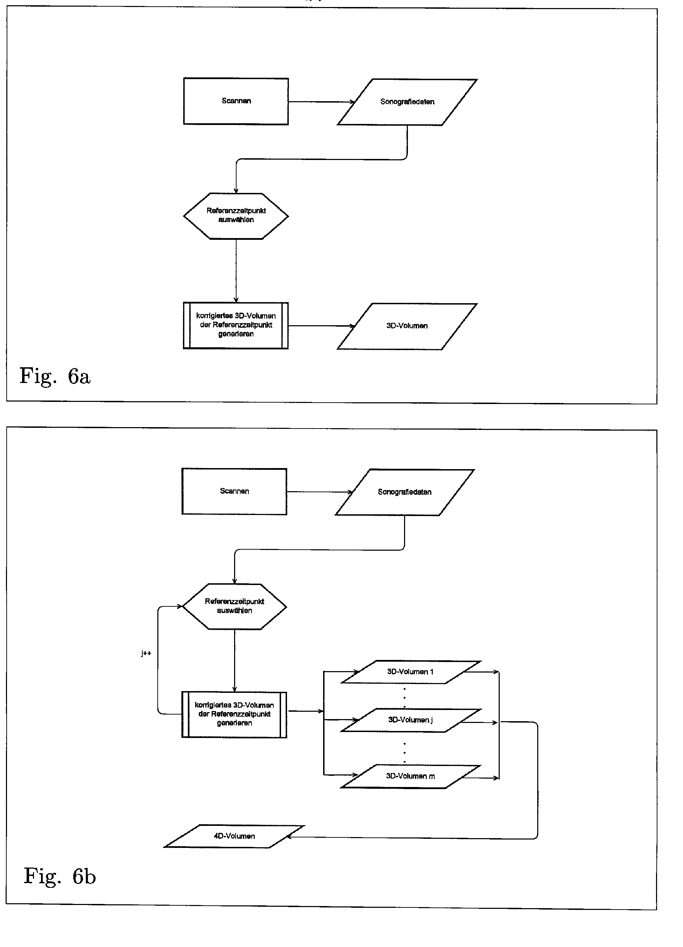

Die Figur 6a zeigt das erfindungsgemäße Verfahren in einem Flussdiagramm. Sonografiedaten werden durch Scannen erzeugt (Figur 6a); wie in Figur 2 wird ein Referenzzeitpunkt ausgewählt und ein korrigiertes 3D-Volumen zum Referenzzeitpunkt generiert. Dieses Verfahren kann z.B. durch einen einfachen Scan-Schwung erzeugt werden. Die Figur 6b erweitert das Verfahren entsprechend Figur 4. Es werden für mehrere Referenzzeitpunkte jeweils Volumen generiert und diese zu einem 4D-Volumen zusammengesetzt.

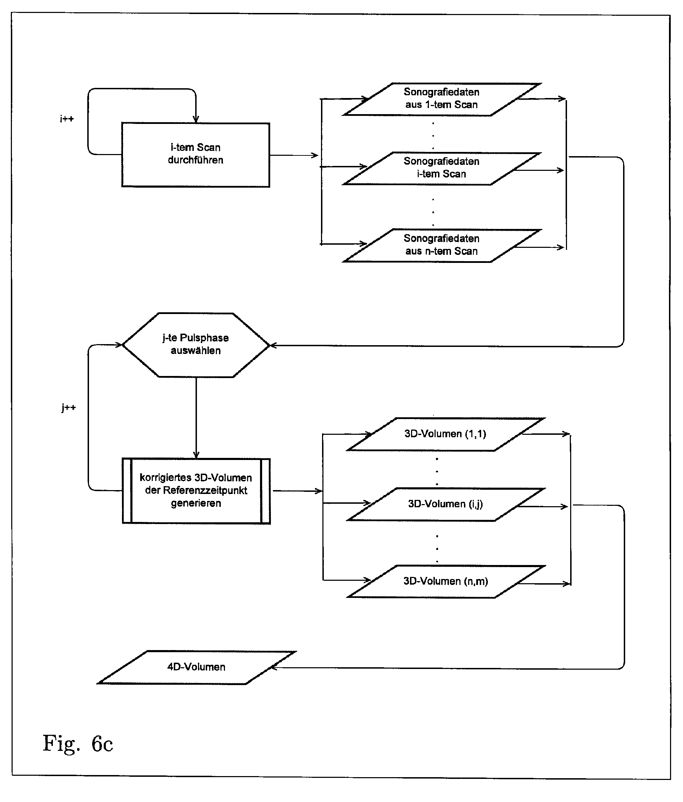

Figur 6c beschreibt eine Aufnahme über mehrere Scan-Schwünge entsprechend Figur 3.

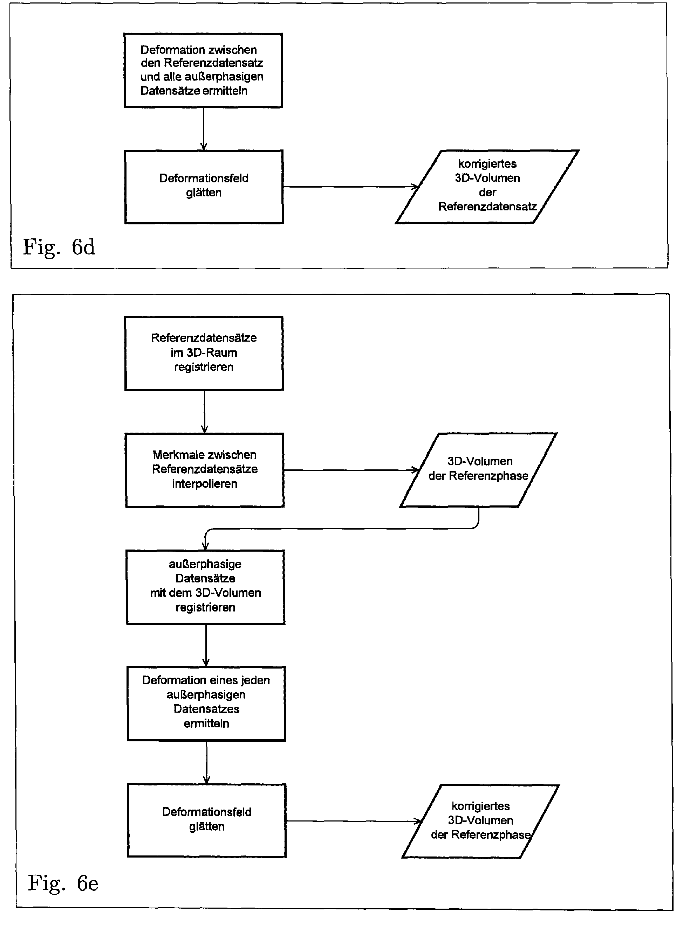

Figur 6d beschreibt, dass mit einer Erweiterung auf den Einsatz von Datensätzen zu mehreren Zeitpunkten oder Phasen die Deformationsfelder zwischen einem aus diesen gewählten Datensatz zum Referenzzeitpunkt oder zur Referenzphase und der anderen Datensätze die zugehörigen Deformationsfelder entlang der Zeitachse geglättet werden.

Figur 6e beschreibt den Vorgang wie in Figur 2: Zwischen verschiedenen Referenzdatensätzen wird ein 3D-Volumen rekonstruiert. Datensätze aus anderen Phasen werden dazu registriert, die Deformation ermittelt, dieses geglättet und ein korrigiertes 3D-Volumen erzeugt.

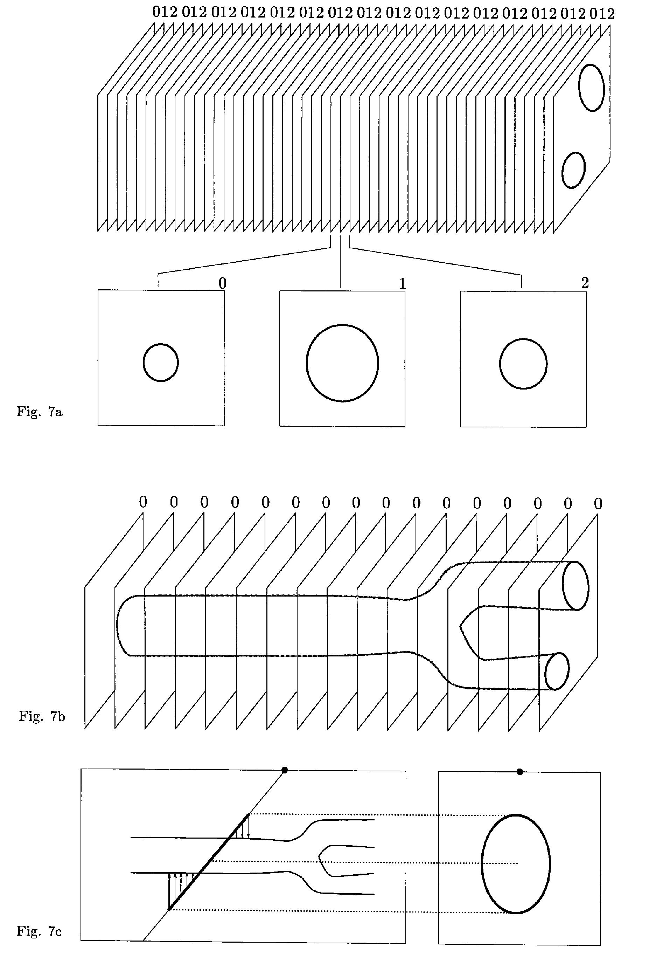

Figur 7 zeigt schematisch eine bevorzugte Ausführungsform des erfindungsgemäßen Verfahrens für den beispielhaften Fall eines Blutgefäßes. Im dargestellten Beispiel weisen die Sonografiedaten von dem dynamischen Objekt, nämlich dem Blutgefäß, drei Datensätze auf, die wie in Figur 7a zu sehen ist, alle drei räumlich überlappen. Der erste Datensatz umfasst dabei Sonografiedaten zu einer ersten Phase 0, der zweite Datensatz umfasst Sonografiedaten zu einer zweiten Phase 1 und der dritte Datensatz umfasst Sonografiedaten zu einer dritten Phase 2. Jeder der drei Datensätze umfasst dabei Sonografiedaten mehrerer Zeitpunkte, aber ein und derselben Phase. Nichtsdestotrotz unterscheiden sich auch die Zeitpunkte der drei Sonografiedatensätze voneinander. Wie schematisch in Figur 7a dargestellt ist, hat das Blutgefäß in der ersten Phase 0 den geringsten Durchmesser, in der zweiten Phase 1 den größten Durchmesser und in der dritten Phase 2 einen mittleren Durchmesser.

Die in Figur 7a schematisch dargestellten Sonografiedaten können dabei auf unterschiedliche Weise gewonnen worden sein. So kann es sich beispielsweise um drei dreidimensionale Datensätze zu den unterschiedlichen Phasen handeln. Alternativ könnte es sich um einen sogenannten "2D sweep" handeln, bei dem zeitlich nacheinander 2D-Datensätze gewonnen werden und der 2D-Schallkopf während der dargestellten Perioden am Gefäß entlang bewegt wird. Die Art und Weise der Datengewinnung ist jedoch für das erfindungsgemäße Verfahren unerheblich, da auch 2D-Datensätze beliebig zu dreidimensionalen Datensätzen zusammengesetzt werden können. Der Begriff "Zeitpunkt" ist in diesem Zusammenhang weit auszulegen, da Sonografiedaten generell durch ein Abtasten gewonnen werden und streng genommen nie alle zu exakt demselben Zeitpunkt aufgenommen werden. Vielmehr entspricht der Begriff "Zeitpunkt", wie er im Rahmen der vorliegenden Erfindung verwendet wird, im Wesentlichen einem Zeitintervall, über das hinweg die Aufnahme erfolgt.

Erfindungsgemäß wird nun mindestens eine Markierung in allen drei Datensätzen ermittelt und eine Bewegung und/oder Deformation bestimmt, die die Markierung von einem Datensatz in die Markierung in einem anderen Datensatz transformiert. Bevorzugt wird im dargestellten Beispiel die Gefäßwand als Markierung dienen. Die Deformation, die die Markierung von einem Datensatz in die Markierung in einem anderen Datensatz transformiert, ist dann im Wesentlichen ein Aufweiten und Zusammenziehen des mit der Gefäßwand korrespondierenden kreisähnlichen Gebildes. Aus dieser Deformation des kreisähnlichen Gebildes kann dann eine Deformation des Blutgefäßes ermittelt werden, was das Ableiten einer Sonografiedatenkorrektur erlaubt. Diese kann beispielsweise im linken Abschnitt des dargestellten Blutgefäßes mithilfe einer Deformationsmatrix dargestellt werden. Im rechten Abschnitt des verzweigten Blutgefäßes ist es möglicherweise notwendig, für jedes Teilgefäß eine separate Deformationsmatrix, oder in einer anderen Ausführungsform eine kombinierte Deformationsmatrix zu bestimmen. Im dargestellten Beispiel mit drei Datensätzen kann dann eine Referenzphase bestimmt werden, die bevorzugt der ersten, zweiten oder dritten Phase entspricht, und ein korrigiertes Sonogramm des Blutgefäßes unter Verwendung der drei Datensätze sowie der abgeleiteten Sonografiedatenkorrektur für diese Referenzphase erstellt werden. Dies lässt sich grundsätzlich für jede der drei Phasen 0, 1 und 2 durchführen, wobei jeweils die beiden anderen Datensätze entsprechend korrigiert werden, sodass letztendlich ein korrigiertes Sonogramm für jede der drei Phasen vorliegt. Die dicke Linie in der linken Darstellung von Figur 7c zeigt die Schnittebene der rechten Grafik. Die senkrechte Achse beider Abbildungen ist identisch, die waagrechten Achsen stehen rechtwinklig zueinander und zur senkrechten Achse. Ein Gefäß aus einer anderen Phase in der rechten Grafik, welches schräg zur Mittellinie des Gefäßes aufgenommen wurde, wird in der linken Grafik auf die Referenzphase deformiert.

Grundsätzlich können die erfindungsgemäß bereitgestellten Sonografiedaten auf unterschiedliche Weise gewonnen werden. Zum einen können sie mit 3D- oder 2D-Schallköpfen aufgenommen werden. Zum anderen kann derselbe Bereich mit einem einzelnen Schwung oder mit mehreren Schwüngen gescannt werden. Schließlich kann es sich um eine periodische oder nicht-periodische Bewegung und/oder Deformation handeln. Daraus ergeben sich die folgenden sechs Kombinationen der Sonografiedatengewinnung (wobei der Einfachheit halber nur von Deformation die Rede ist):
1. 3D Schallkopf - stationär
   a. Nichtzyklische Deformation - z. B. Bewegung des Patienten oder weniger Scanzeit als ein Periode (beispielsweise ein Herzschlag oder kürzer)
   b. Zyklische Deformation - mehrere Perioden (beispielsweise mehrere Herzschläge)
2. 3D Schallkopf - über die interessierende Region bewegt, zyklische Deformation
   a. Einzelner Scan-Schwung
   b. Mehrfache Scan-Schwünge
3. 2D Schallkopf (getrackt) - über die interessierende Region bewegt, zyklische Deformation
   a. Einzelner Scan-Schwung
   b. Mehrfache Scan-Schwünge

Im Falle eines stationären 3D Schallkopfes und einer nicht-periodischen Deformation (vgl. Fig. 1) kann das erfindungsgemäße Verfahren beispielsweise folgendermaßen durchgeführt werden: Der 3D Schallkopf generiert eine zeitgeordnete Reihe von 3D Ultraschall-Datensätzen (*Vi*,*ti*)⊂R³×[0,∞) mit *i*∈{0,1,...,*N*} des im Wesentlichen selben Raumbereichs. Sei *V*=∩*ⁿ*_{*i*=0}*Vi* der räumliche Durchschnitt aller 3D Datensätze. Im folgenden nehmen wir an, *dass Vi*∩*V= Vi,*∀*i.*

Nun wird ein Datensatz zu einer Referenzzeit als Referenzdatensatz ausgewählt, zum Beispiel *iref=*0*.* Sei *κᵢ*, die Deformation vom Referenzdatensatz zum *i^{ten}* Datensatz, so dass *κᵢ*(*V_{ref})=Vi.* Dann wird die Deformation zwischen dem Referenzdatensatz und jedem anderen Datensatz, d.h. *κᵢ* für alle *i≠iref,* bestimmt, indem die Deformation mindestens einer Markierung zwischen den einzelnen Datensätzen ermittelt wird. Bei diesen Markierungen handelt es sich, wie oben beschrieben, z. B. um geometrische und/oder anatomische Strukturen innerhalb der Datensätze.

Aus dieser Deformation zwischen den Datensätzen wird der Deformationsgradient *Fi* für jede Deformation *κi* berechnet. Aus der Gesamtheit der Deformationgradienten Fi kann ein über Raum und Zeit geglättetes tensorielles Deformationsfeld abgeleitet werden. Anhand dieses Deformationsfeldes kann dann jeder beliebige Datensatz zu einem beliebigen anderen Datensatz optimal deformiert bzw. korrigiert werden. Das heißt, die Information von allen anderen Datensätzen kann benutzt werden, um die Bildqualität und die Information eines beliebigen Datensatzes zu verbessern. Bevorzugt wird dieses Verfahren für alle gegebenen Datensätze durchgeführt, so dass für jeden Zeitpunkt ein optimiertes Sonogramm erstellt werden kann.

Das führt zu einer zeitgeordneten Reihe von verbesserten 3D Ultraschall-Datensätzen, die beispielsweise kombiniert werden können, um einen optimierten 4D Datensatz zu generieren und/oder darzustellen.

Im Falle einer zyklischen Deformation (Fall 1.b) nehmen wir an, dass die Deformation periodisch mit Periode *T* ist. Das heißt: *V(t+nT)=V(T)* ∀*n*∈{0,1,2,...}.

Falls die Frequenz der Generierung der Datensätze (d.h. Volumen pro Sekunde) ein ganzzahliges Vielfaches der Deformationsfrequenz ist, also *fdata=p·*1/*T* für eine nichtnegative ganze Zahl p, dann korrespondieren die Datensätze, die zu einer bestimmten Phase *ϕ*∈[0,*T*) erstellt werden, genau mit derselben Phase jedes Zyklus. D.h. man erhält genau p Datensätze pro Zyklus, die zeitlich genau übereinstimmen. Es ist jedoch extrem unwahrscheinlich, dass dies zufällig geschehen würde. Im folgenden nehmen wir daher an, dass das nicht der Fall ist.

Dies führt dann dazu, dass beim Scan mehrerer Perioden eine Phasenverschiebung zwischen Scan-Phase und Deformations-Phase auftritt, so dass bei der Generation eines optimierten 4D Datensatzes eine höhere Zeitauflösung erzielt werden kann als in dem nicht-periodischen Fall. Im Falle eines bewegten 3D Schallkopfes (Fälle 2.a und 2.b) wird der Schallkopf bewegt, um ein größeres Volumen zu scannen. Dabei müssen zwei zeitlich benachbarte Datensätze nicht unbedingt räumlich überlappen. Der räumliche Überlapp kann vielmehr auch zwischen anderen Paaren von Datensätzen bestehen, etwa aufgrund eines mehrmaligen Scannens derselben Region. Das Ziel ist, so viel des Raumzeitbereichs Ω×[0,*T*) wie möglich abzudecken, wobei Ω⊂R³ die Region von Interesse ist. Das kann man erreichen wenn man entweder i) die Region langsam mehrmals kontinuierlich scannt, oder ii) wenn man den Schallkopf stationär hält für (mindestens) einen Deformationszyklus, und danach zu einer benachbarten Subregion für (mindestens) einen Deformationszyklus wechselt, bis die ganze interessierende Region abgedeckt ist.

Da hier im Fall i) der Schallkopf während des Zyklus nicht stationär ist, erhält man im allgemeinen keine zwei Datensätze zweier verschiedener Phasen, die vollständig räumlich überlappen. In diesem Fall wird die Deformation nur für die Durchschnittsregion der beiden Datensätze berechnet. Wie im Fall 1.a wird diese Deformation bevorzugt für alle Datensätze berechnet und die einzelnen Deformationen werden kombiniert, um ein 4D Deformationsfeld zu erstellen. Unter Verwendung dieses Deformationsfelds werden die 3D Datensätze einzeln verbessert und anschließend kombiniert, um einen 4D Datensatz der ganzen interessierenden Region zu generieren, wie dies in Fig. 5 schematisch dargestellt ist.

Alternativ kann zunächst ein ergänzter 3D Datensatz aus allen 3D Datensätzen einer bestimmten Phase *ϕ*∈[0,*T*) aller Perioden erzeugt werden und danach die Deformationen zwischen den ergänzten 3D Datensätzen verschiedener Phasen bestimmt werden, wie dies schematisch in den Figuren 2 und 4 dargestellt ist.

Im Falle eines 2D Schallkopfes (Fälle 3.a und 3.b) wird der (möglicherweise getrackte) 2D Schallkopf über die interessierende Region bewegt. Die Schallkopftrajektorie kann beliebig sein, aber das Ziel ist wie vorher, so viel des Raumzeitbereichs Ω×[0,*T*) wie möglich abzudecken. Die Periode der zyklischen Bewegung wird bevorzugt in diskrete Intervalle aufgeteilt. Jedes Intervall korrespondiert mit einer bestimmten Phase *ϕ*∈[0,*T*) des Zyklus. Analog zur Technologie des Gating (die z. B. bei der CT- und MRI-Technologie Anwendung findet), werden die 2D Datensätze entsprechend der Phase der Deformation, zu der der Datensatz generiert wurde, gruppiert. Jede Phasengruppe wird entweder durch Tracking oder durch bildbasierte Registrierungsverfahren räumlich registriert. Davon wird ein 3D Datensatz erzeugt (vgl. *Three-dimensional ultrasound imaging* Fenster et al., 2001). Dieses Verfahren wird für jedes Phasenintervall durchgeführt. Dadurch erhält man eine Vielzahl von 3D Datensätze für eine endliche Anzahl von Phasen über die ganze Periode des Zyklus. Anschließend wird die Deformation zwischen den räumlich überlappenden Regionen der 3D Datensätze wie im Grundfall 1.a bestimmt.

Die hier beschriebenen Ausführungsformen sind lediglich beispielhaft zu verstehen. Der Grundgedanke der vorliegenden Erfindung wird letztlich durch die Art der Datengewinnung und die dadurch verursachten Auswertungsvarianten nicht tangiert. Vielmehr ändert dies lediglich die Art der anspruchsgemäß bereitgestellten Daten. So beginnt in den Fällen 3.a und 3.b die erfindungsgemäße Auswertung im Prinzip mit den Datensätzen bestimmter Phasen.

## Patentansprüche

1. Verfahren zur Auswertung von Sonografiedaten zur verbesserten Darstellung eines dynamischen Objektes, wobei das Verfahren die folgenden Schritte aufweist:
a) Bereitstellen von Sonografiedaten von einem dynamischen Objekt, wobei die Sonografiedaten mindestens einen ersten und einen zweiten Datensatz aufweisen, wobei der erste Datensatz und der zweite Datensatz zumindest teilweise räumlich überlappen und wobei der erste Datensatz mindestens Sonografiedaten zu einem ersten Zeitpunkt t₁ umfasst und der zweite Datensatz mindestens Sonografiedaten zu einem zweiten, vom ersten Zeitpunkt verschiedenen Zeitpunkt t₂ umfasst;
b) Bestimmen mindestens einer Markierung im ersten und zweiten Datensatz und Ermitteln einer Bewegung und/oder Deformation, die die Markierung im ersten Datensatz in die Markierung im zweiten Datensatz transformiert;
c) Ermitteln einer Bewegung und/oder Deformation des Objektes auf Basis der ermittelten Bewegung und/oder Deformation der mindestens einen Markierung;
d) Ableiten einer Sonografiedatenkorrekur auf Basis der ermittelten Bewegung und/oder Deformation des Objektes; und
e) Erstellen eines korrigierten Sonogramms des Objektes unter Verwendung des ersten und zweiten Datensatzes sowie der abgeleiteten Sonografiedatenkorrektur.

2. Verfahren nach Anspruch 1, wobei die mindestens eine Markierung eines oder eine Kombination der folgenden Elemente aufweist: einen oder mehrere Markierungspunkte, eine oder mehrere Markierungskanten, eine oder mehrere Markierungsflächen, eine oder mehrere Grenzflächen, insbesondere Grenzflächen zwischen unterschiedlichen Materialien des Objektes, eine oder mehrere Oberflächen von Teilobjekten des Objektes, anatomische Merkmale des Objektes, absolute und/oder relative Intensitätswerte und/oder Frequenzspektrum der Intensitätswerte mindestens eines Teils der Sonografiedaten.

3. Verfahren nach einem der vorhergehenden Ansprüche, wobei das Ableiten der Sonografiedatenkorrektur aufweist: Bestimmen mindestens eines Verschiebevektors mindestens eines Markierungspunktes und/oder Bestimmen mindestens einer Deformationsmatrix und/oder Bestimmen eines geglätteten Deformationsfeldes.

4. Verfahren nach einem der vorhergehenden Ansprüche, wobei das Erstellen des Sonogramms derart erfolgt, dass der erste und/oder zweite Sonografiedatensatz korrigiert wird und ein Sonogramm des Objektes zu einem Referenzzeitpunkt, bevorzugt aus dem Zeitintervall [t₁, t₂], erstellt wird.

5. Verfahren nach Anspruch 4, wobei das Verfahren für mehrere Referenzzeitpunkte durchgeführt wird.

6. Verfahren nach einem der vorhergehenden Ansprüche, wobei die Sonografiedaten von einem zyklisch bewegten und/oder deformierten Objekt stammen und wobei der erste Datensatz Daten einer ersten Phase ϕ₁ der zyklischen Bewegung und/oder Deformation umfasst und der zweite Datensatz Daten einer zweiten, von der ersten Phase verschiedenen Phase ϕ₂ der zyklischen Bewegung und/oder Deformation umfasst.

7. Verfahren nach Anspruch 6, wobei das Erstellen des Sonogramms derart erfolgt, der erste und/oder zweite Sonografiedatensatz korrigiert wird und ein Sonogramm des Objektes zu einer Referenzphase, bevorzugt aus dem Phasenintervall [ϕ₁, ϕ₂], erstellt wird.

8. Verfahren nach Anspruch 7, wobei das Verfahren für mehrere Referenzphasen durchgeführt wird.

9. Verfahren nach einem der Ansprüche 6 bis 8, wobei der erste Datensatz Daten unterschiedlicher Zeiten, aber derselben Phase umfasst und/oder zweite Datensatz Daten unterschiedlicher Zeiten, aber derselben Phase umfasst.

10. Verfahren nach einem der Ansprüche 6 bis 9, ferner mit dem Schritt der Bestimmung der Phase der zyklischen Bewegung und/oder Deformation, wobei die Phase auf Basis der Sonografiedaten und/oder mit Hilfe einer zusätzlichen Messeinheit bestimmt wird.

11. Verfahren nach einem der Ansprüche 7 bis 10, wobei die zyklische Bewegung und/oder Deformation durch Puls und/oder Atmung definiert wird.

12. Verfahren nach einem der vorhergehenden Ansprüche, wobei die Datenqualität der Sonografiedaten ermittelt wird und mindestens ein Teil der Sonografiedaten eines oder mehrerer Zeitpunkte bzw. einer oder mehrerer Phasen schlechter Datenqualität verworfen wird.

13. Verfahren nach einem der vorhergehenden Ansprüche, wobei mehrere Sonogramme zu unterschiedlichen Zeitpunkten oder Phasen erstellt werden und, bevorzugt zeitlich nacheinander, angeordnet werden.

14. Computerprogramm zur Durchführung des Verfahrens nach einem der Ansprüche 1 bis 13.

15. Ultraschallgerät mit einer Signalprozessoreinheit, wobei das Ultraschallgerät dazu geeignet ist, Sonografiedaten von einem dynamischen Objekt aufzunehmen, wobei die Sonografiedaten mindestens einen ersten und einen zweiten Datensatz aufweisen, wobei der erste Datensatz und der zweite Datensatz zumindest teilweise räumlich überlappen und wobei die Signalprozessoreinheit dazu geeignet ist, die Schritte b) bis e) gemäß Anspruch 1 und optional die Schritte der Ansprüche 2 bis 13 durchzuführen, wobei das Ultraschallgerät bevorzugt eine Einrichtung zum Messen von Puls und/oder Atmung aufweist.
